(19) Europäisches Patentamt
European Patent Office
Office européen des brevets

(11) **EP 3 778 800 A1**

(12) **EUROPEAN PATENT APPLICATION**
published in accordance with Art. 153(4) EPC

(43) Date of publication:
**17.02.2021 Bulletin 2021/07**

(51) Int Cl.:
*C09D 11/328* [(2014.01)]   *B41J 2/01* [(2006.01)]
*B41M 5/00* [(2006.01)]   *B41M 5/52* [(2006.01)]
*C09B 11/26* [(2006.01)]   *C09B 29/16* [(2006.01)]
*C09B 67/46* [(2006.01)]

(21) Application number: **19781618.4**

(22) Date of filing: **03.04.2019**

(86) International application number:
**PCT/JP2019/014881**

(87) International publication number:
**WO 2019/194254 (10.10.2019 Gazette 2019/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.04.2018 JP 2018072468**

(71) Applicant: **Toppan Printing Co., Ltd.**
**Tokyo 110-0016 (JP)**

(72) Inventors:
• **ISHIKAWA Hideki**
**Tokyo 110-0016 (JP)**
• **HOSHINO Yuichi**
**Tokyo 110-0016 (JP)**
• **SAITO Masatoshi**
**Tokyo 110-0016 (JP)**

(74) Representative: **TBK**
**Bavariaring 4-6**
**80336 München (DE)**

(54) **BLUE INKJET INK AND TABLET**

(57)    The present invention aims to provide a blue ink-jet ink that improves lightfastness of the same while maintaining its blue print color and a tablet including a part printed with the blue ink-jet ink. The blue ink-jet ink according to the present embodiment is an edible ink-jet that contains Brilliant Blue FCF (Blue No. 1 Colorant) and New Coccine (Red No. 102 Colorant) as colorants.

**Description**

[Technical Field]

The present invention relates to a blue ink-jet ink and a tablet.

[Background Art]

Some inks for ink-jet printing (hereinafter referred to simply as an "ink-jet ink") are edible. An illustrative technique related to an edible ink-jet ink is one described in Patent Literature 1.

Printed characters and images printed in some prior art ink-jet inks undergo photo-fading, i.e., have poor light fastness. An ink-jet ink with poor light fastness may result in poor readability and a change in color tone. Brilliant Blue FCF, or Blue No. 1 Colorant (FDA name: FD & C Blue No. 1, Color index name: Food Blue 2, CAS number: 3844-45-9), which exhibits a vivid blue color, in particular has poor light fastness. An ink-jet ink containing the colorant may therefore possibly cause color dullness or loss of printing.

[Citation List]

[Patent Literature]

[PTL 1] JP 2006-169301 A

[Summary of the Invention]

[Technical Problem]

The present invention has been made in view of the circumstances described above and aims to provide a blue ink-jet ink that improves the light fastness thereof while keeping its print color blue and a tablet including a part printed in the blue ink-jet ink.

[Solution to Problem]

The blue ink-jet ink according to one aspect of the present invention is an edible ink-jet ink that contains Brilliant Blue FCF and New Coccine as colorants.

[Advantageous Effects of the Invention]

An aspect of the present invention improves the lightfastness of an ink-jet ink while keeping its print color blue.

[Brief Description of the Drawings]

Fig. 1 is a conceptual view illustrating the lightfastness of an ink-jet ink according to an embodiment of the present invention.
Fig. 2 is a schematic cross-sectional view illustrating an example of a tablet (uncoated tablet) according to an embodiment of the present invention.
Fig. 3 is a schematic cross-sectional view illustrating an example of a tablet (film-coated tablet) according to an embodiment of the present invention.
Fig. 4 is an example of a printed image on a tablet (uncoated tablet) according to an embodiment of the present invention.
Fig. 5 is an example of a printed image on a tablet (film-coated tablet) according to an embodiment of the present invention.
Fig. 6 shows relationships between a ratio of Brilliant Blue FCF to a total amount of colorants contained in an ink-jet ink and a degree of photo-fading.
Fig. 7 shows relationships between a ratio of a total mass of Brilliant Blue FCF and New Coccine to a total mass of an ink-jet ink, and a degree of photo-fading.
Fig. 8 illustrates criteria for evaluating print resumability in an example of the present invention.

[Detailed Description]

An ink-jet ink according to an embodiment of the present invention relates to an ink-jet ink that improves lightfastness of an image or the like printed on a surface of a medical tablet by an ink-jet printing method. A detailed description will be given of an ink-jet ink according to an embodiment of the present invention (hereinafter also referred to simply as a "blue ink-jet ink") and a composition of a tablet having a part printed with the blue ink-jet ink.

[Composition of a blue ink-Jet ink]

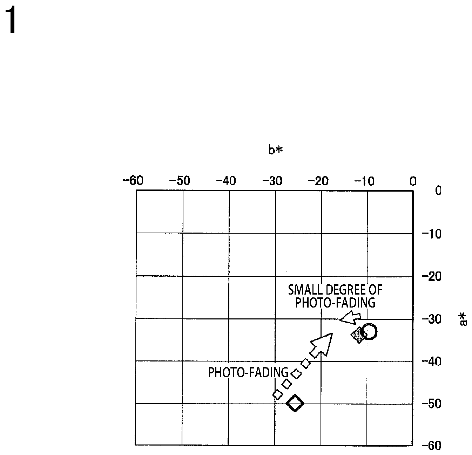

A blue ink-jet ink according to the present embodiment is an ink having a color tone defined as follows.
First, a non-overlapped solid image is created on a print target at an amount of 12 pl per drop using a piezoelectric ceramic driven drop-on-demand inkjet head having a printing resolution of 600 dpi in a main direction, 600 dpi in a sub-scanning direction (direction in which a tablet and other print target medium are conveyed), and a total nozzle count of 2,656.
A spectral densitometer "X-Rite 530", which is manufactured by X-Rite, Inc. is then placed above the printed solid image to measure an optical density of cyan, magenta, and yellow when a color tone (an L*a*b* color system) of reflected light and a print color estimated from the reflected light are expressed with these colors. These spectral densities are measured at a viewing angle of 2 degrees and under a light source D50.
Among the color tones expressed in the L*a*b* color system measured under the above-mentioned conditions, a color (color tone) that is in the range of

$$b* \leq -5$$

is defined as "blue" in the present invention, and a color (color tone) that is in the range of

$$b* \leq -20$$

is defined as "vivid blue" in the present embodiment.
In addition, an optical density of cyan (C value) is used this time to evaluate the blue print. If the C value is 0.5 or more, it is assumed that the blue has a sufficient optical density for visual recognition.
Note that a preferred print target is, for example, a tablet having a white surface (70 degrees or more whiteness). The whiteness can be calculated from a spectrum of reflected light measured with an optical densitometer "X-Rite eXact", which is manufactured by X-Rite Inc., placed above a substrate such as a tablet or a sheet of paper. The setup conditions comply with TAPPI - T452, which is determined by the American Paper & Pulp Association (TAPPI). Other than a tablet, a sheet of paper can be also used as a print target as long as it is of a whiteness that is 70 degrees or more.
The blue ink-jet ink according to the present embodiment invention is an edible ink-jet ink that contains Brilliant Blue FCF (also referred to as Blue No. 1) (FDA name: FD & C Blue No. 1, Color index name: Food Blue 2, CAS number: 3844-45-9) and New Coccine (also referred to as Red No. 102) (Color index name: Acid Red 18, CAS number: 2611-82-7). Such a composition improves the lightfastness of an ink-jet ink while keeping its print color blue. A description will be given below of this point with reference to Fig. 1.
The "Color Index Name" mentioned above is determined by the American Association of Textile Chemists and Colorists. The "FDA Name" mentioned above is determined by the FDA (U.S. Food and Drug Administration). Each available colorant (each material) is identified with a CAS Number in the present embodiment but the present invention is not limited thereto. For example, a colorant (substance) that is named identically to a colorant (substance) described in the present embodiment but carries a different CAS number because it is a substance containing a geometric isomer, a stereoisomer element, or an isotope element or is salt made therefrom may also be used in the present embodiment as a matter of course. If an isomer or the like is not present or an available colorant (substance) is specified (limited), the substance (compound) with the CAS number described in the present embodiment can be used.
Fig. 1 is a conceptual view illustrating the lightfastness of an ink-jet ink according to an embodiment. The "0" symbol shown in Fig. 1 represents a color tone of Brilliant Blue FCF before photo-fading, while the "♦" symbol represents a color tone of Brilliant Blue FCF after photo-fading. The vertical axis of Fig. 1 represents "a*" values in the L*a*b* color system. The horizontal axis of Fig. 1 represents "b*" values in the L*a*b* color system.
As shown in Fig. 1, the Brilliant Blue FCF used in the present embodiment is denatured by light, changing its color tone in the direction of the arrow (dashed line). The value of the change in color tone exceeding a certain numeric value can be considered as "photo-fading". In other words, Brilliant Blue FCF photo-fades in the direction of the arrow (dashed line) shown in Fig. 1.

In the present embodiment, New Coccine is added to the ink-jet ink containing Brilliant Blue FCF to lower the chroma of the ink color tone in advance along a direction in which Brilliant Blue FCF photo-fades. This is in expectation of reducing an amount of change in the color tone of the Brilliant Blue FCF between before and after the photo-fading, resulting in improvement in the ink lightfastness. Fig. 1 shows an example of the color of a mixed ink of Brilliant Blue FCF and New Coccine with a "o"symbol, where the Brilliant Blue FCF is added along a direction in which photo-fading occurs.

The blue ink-jet ink according to the present embodiment may have a mass ratio of Brilliant Blue FCF to New Coccine in the range of 1:0.025 to 1:2. Such a composition further improves the lightfastness of the ink-jet ink while maintaining its blue print color. Amass ratio of Brilliant Blue FCF to New Coccine ranging from 1:0.025 to 1:0.43 can keep the print color as a vivid blue while keeping the color lightfast.

In the blue ink-jet ink according to the present embodiment, the total mass of colorants including Brilliant Blue FCF and New Coccine may be in the range of 1 mass% or more and 18 mass% or less of the whole blue ink-jet ink. Such a composition improves the lightfastness of the ink-jet ink while maintaining its blue print color blue, and additionally provide the ink with high print resumability. A total amount of colorants below 1 mass% of the whole blue ink-jet ink is so low that the optical density (C value) of blue print will be less than or equal to 0.5, which is a reference value that is deemed to be sufficient for the blue print to be recognized as blue, which in turn tends to lower both readability and lightfastness of the ink. Conversely, a total mass of colorants over 18 mass% of the whole blue ink-jet ink is so high that the print resumability tends to drop.

Now the effects described above will be detailed below.

Brilliant Blue FCF and New Coccine are both used as an ink-jet ink colorant. Using Brilliant Blue FCF alone as an ink-j et ink colorant for medical tablets or the like, however, fails to provide a printed image with sufficient lightfastness. Specifically, a degree of fading ΔE of a solid print image is approximately 34 in, for example, a sunlight exposure test of 1.2 million lux. This is a circumstance in which fading of a printed character can be visually observed.

In the present embodiment, a sufficient lightfastness with which to form an image to be printed on a medical tablet or the like refers mostly to a degree of fading ΔE that is less than or equal to 23 before and after a solid print image is exposed to light in a solar radiation exposure test of 1.2 million lux. This is a result derived from an opinion test carried out by the inventors together with medical practitioners, where a change in color becomes easier to be recognized when ΔE exceeds 15, and the printed image begins to be visually recognized to be less readable when the ΔE exceeds 23. These figures are larger than a standard degree of fading ΔE in general commercial printed matter, which ranges from 3 to 6, but this is because no comparison is made on the tablet before and after the photo-exposure and the tablet is assumed to fade by itself. Note that the fading refers to a degree of change in color tone due to the action of light.

In the present embodiment, Brilliant Blue FCF should contain New Coccine at at least 0.025 parts by mass relative to 1 part by mass of Brilliant Blue FCF because a smaller amount (e.g., 0.001 part by mass) of New Coccine relative to 1 part by mass of Brilliant Blue FCF causes ΔE to be over 23 when a lightfastness test is carried out on a solid printed image. Increasing the amount of New Coccine gradually lowers ΔE. Increasing the amount of New Coccine to more than 0.025 part by mass relative to 1 part by mass of Brilliant Blue FCF lowers ΔE below 23, resulting in higher lightfastness than that of blue ink-jet ink in which Brilliant Blue FCF is used alone.

In addition, New Coccine of up to 2 parts by mass may be blended with 1 part by mass of Brilliant Blue FCF in the present embodiment because blending a larger amount of New Coccine (e.g., 3 part by mass relative to 1 part by mass of Brilliant Blue FCF) causes the chromaticity indicative of blue to be greater than -5, resulting in an ink that fails to appear blue.

The present embodiment exhibits an effect presumably because Brilliant Blue FCF fades in the L*a*b* color system in a direction at a time of fading that coincides with the direction in which the Brilliant Blue FCF blended with New Coccine fades in the L*a*b* color system. The Brilliant Blue FCF mixes with another portion of Brilliant Blue FCF that has not photo-faded and exhibits an identical color and New Coccine at a time of photo-fading, presumably thereby reducing an apparent change in color.

In other words, New Coccine is preferably blended with Brilliant Blue FCF preferably at the part by mass ratio of 0.025 to 2:1 in the ink-jet ink of the present embodiment, and more preferably at the ratio of 0.025 to 0.43:1.

In addition, the whole ink preferably contains a colorant including a total content of Brilliant Blue FCF and New Coccine in the range of 1 mass% or more and 18 mass% or less. A content below 1 mass% provides poor print density. A content above 18 mass%, on the other hand, prevents the colorant from stably dissolving, which in turn precipitates the colorant out of the ink as a solid, possibly clogging the inkjet head nozzle, i.e., lowering so-called print resumability.

(Colorant)

A colorant contained in the blue ink-jet ink according to the present embodiment includes Brilliant Blue FCF and New Coccine as described above, but a colorant other than these two is subject to no particular limitation as long as it is edible. A colorant that can be added to the blue ink-jet ink according to the present embodiment may be selected as necessary from, for example, conventionally known synthetic and natural edible colorants.

Examples of the synthetic edible colorant include a tar colorant, a natural colorant derivative, and a natural synthetic colorant. Examples of the tar colorant include edible Red No. 2 (Amaranth, FDA name: FD & C Red No.2, Color index name: Acid Red 27, CAS number: 915-67-3), edible Red No. 3 (Erythrosine, FDA name: FD&C Red No. 3, Color index name: Acid Red 51, CAS number: 16423-68-0), edible Red No. 40 (Allura Red AC, FDA name: FD & C Red No. 40, Color index name: Food Red 17, CAS number: 25956-17-6), edible Red No. 104 (Phloxine B, FDA name: D & C Red No.28, Color index name: Acid Red 92, CAS number: 18472-87-7), edible Red No. 105 (Rose Bengal, Color index name: Acid Red 94, CAS number: 632-69-9), edible Red No. 106 (Acid Red, Color index name: Acid Red 52, CAS number: 3520-42-1), edible yellow No. 4 (Tartrazine, FDA name: FD & C Yellow No.5, Color index name: Acid Yellow 23, CAS number: 1934-21-0), edible yellow No. 5 (Sunset Yellow FCF, FDA name: FD & C Yellow No.6, Color index name: Food Yellow 3, CAS number: 2783-94-0), edible blue No. 2 (Indigo Carmine, FDA name: FD & C Blue No.2, Color index name: Acid Blue 74, CAS number: 860-22-0), edible Red No. 2 aluminum lake (FD & C Red No. 2 Aluminum Lake), edible Red No. 3 aluminum lake (FD & C Red No.3 Aluminum Lake), edible Red No. 40 aluminum lake (FD & C Red No.40 Aluminum Lake), edible yellow No. 4 aluminum lake (FD & C Yellow No. 5 Aluminum Lake), edible yellow No. 5 aluminum lake (FD & C Yellow No.6 Aluminum Lake), edible Blue No. 1 aluminum lake (FD & C Blue No.1 Aluminum Lake), edible Blue No. 2 aluminum lake (FD & C Blue No.2 Aluminum Lake). Examples of the natural colorant derivative include norbixin potassium or the like. Examples of the natural synthetic colorant include β-carotene, riboflavin, or the like.

Examples of the natural edible colorant include an anthocyanin colorant, a carotenoid colorant, a quinone colorant, a chlorophyll colorant, a flavonoid colorant, a betaine colorant, a monascus colorant, and other natural colorants originating from natural products. Examples of the anthocyanin colorant include red radish colorant, red cabbage colorant, red rice colorant, elderberry colorant, cowberry colorant, gooseberry colorant, cranberry colorant, salmon berry colorant, perilla colorant, sim blueberry colorant, strawberry colorant, dark sweet cherry colorant, cherry colorant, hibiscus colorant, huckleberry colorant, grape juice colorant, grape pericarp colorant, black currant colorant, blackberry colorant, blueberry colorant, plum colorant, whortleberry colorant, boysenberry colorant, and mulberry colorant, purple potato colorant, purple corn colorant, purple potato colorant, and raspberry colorant, red currant colorant, loganberry colorant, and other anthocyanin colorant. Examples of the carotenoid colorant include annatto colorant, gardenia yellow colorant, and other carotenoid colorants. Examples of the quinone colorant include cochineal colorant, lithospermi radix colorant, lac colorant, and other quinone colorants. Examples of the flavonoid colorants include safflower yellow colorant, kaoliang colorant, onion colorant, and other flavonoid colorants. Examples of the betaine colorants include beet red colorant. Examples of the monascus colorants include monascus colorant and monascus yellow colorant. Examples of other colorants originating from natural products include turmeric colorant, clerodendrum trichotomum colorant, gardenia red colorant, spirulina blue colorant, or the like.

(Dispersion medium)

In addition to the colorants mentioned above, the blue ink-jet ink according to the present embodiment may contain a dispersion medium to disperse these colorants. Examples of the dispersion medium that can be added to the blue ink-jet ink according to the present embodiment include purified water, ethanol, glycerin, propylene glycol, polyethylene glycol 300 (average molecular weight 300), 1-propanol, 2-propanol, ethyl lactate, or the like. There is no particular limitation on the blending ratio but the ink preferably contains any of glycerin, propylene glycol, or polyethylene glycol 300 in the range of 1 wt% or more and 30 wt% or less to prevent the ink from drying in the nozzle. A content below 1 mass% is liable to cause the ink to dry, possibly causing the nozzle to clog, whereas a content above 30 mass% causes a print surface on the surface of a medical tablet to dry too slowly, possibly causing defects on adjacent tablets such as those due to undried ink when printed tables come into contact with each other.

There is no particular limitation on the medical tablet in the present embodiment, but the ink tends to be more effective on a film-coated tablet coated with a film on a surface thereof. This is presumably because the film-coated tablet has fewer voids on the printed surface than an uncoated tablet, thereby making it more feasible for colorant compositions to remain on the tablet surface, which means that the colorant compositions on the surface of the film-coated tablet are more subject to the external influence of light or humidity. An uncoated tablet tends to be relatively less subject to this influence because it has more voids, which means that the printed surface fades due to permeation of the colorant into the tablet over time.

[Printing method]

The blue ink-jet ink according to the present embodiment places no particular limitation on the printing method and allows printing with an ink-jet device such as a commercially available ink-jet printer. The ink-jet ink according to the present embodiment has therefore a wide range of application and is very useful. The ink-jet ink according to the present embodiment can work with a so-called drop-on-demand inkjet device, which is actuated by piezoelectric elements (piezoelectric ceramics), and other type of ink-jet devices.

Examples of the drop-on-demand ink-jet device include a thermal ink-jet device that ejects ink-jet ink with a vapor pressure generated by instantaneously heating micro heating elements to a high temperature (200 to 300 °C), an electrostatic device that ejects ink-jet ink by electrostatically vibrating an actuator, and an ultrasonic wave device that utilizes a cavitation phenomenon of caused by ultrasonic waves. If the blue ink-jet ink according to the present embodiment is electrically chargeable, a continuously injectable (continuous type) device can also be used.

[Tablet]

In the present embodiment, the blue ink-jet ink according to the present embodiment may be printed, for example, on the surface of a tablet using the printing methods described above. In other words, the blue ink-jet ink according to the present embodiment improves lightfastness of a part of the surface of a medical tablet on which printing is carried out in an inkjet printing method, i.e. of a printed image. A description will be given of a composition of a tablet having an image printed with the blue ink-jet ink according to an embodiment.

The tablet according to the present embodiment is, for example, a medical tablet. The "medical tablet" includes, for example, a film-coated tablet having a water-soluble surface layer formed on the outermost surface thereof as well as an uncoated tablet (naked tablet), a sugar-coated tablet, an enteric tablet, an oral disintegrating tablet and the like.

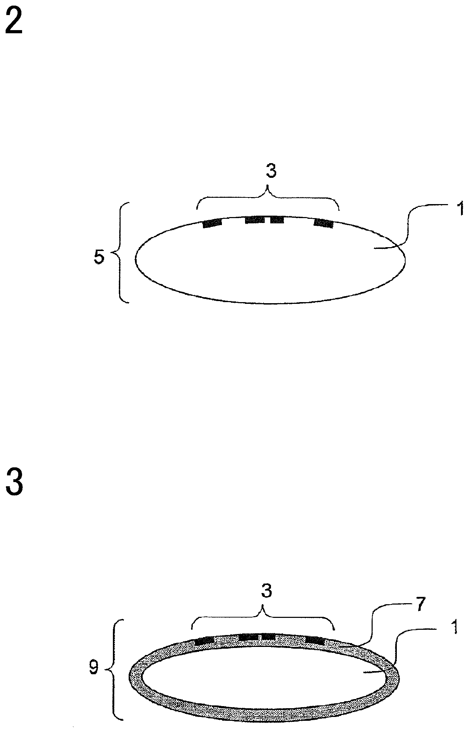

Fig. 2 is a schematic cross-sectional view illustrating an example of a medical tablet (uncoated tablet) on which characters or an image is printed. The figure shows a printed uncoated tablet 5 with a printed image 3, such as characters, printed on an upper surface of a substrate 1 of the tablet in cross-sectional view.

Fig. 3 is a schematic cross-sectional view illustrating an example of a medical tablet (film-coated tablet) on which a letter or an image is printed. The figure shows a printed film-coated tablet 9 with a printed image 3, such as characters, printed on an upper surface of a substrate 1 of the tablet on which a film-coated layer 7 is formed on a surface thereof in cross-sectional view.

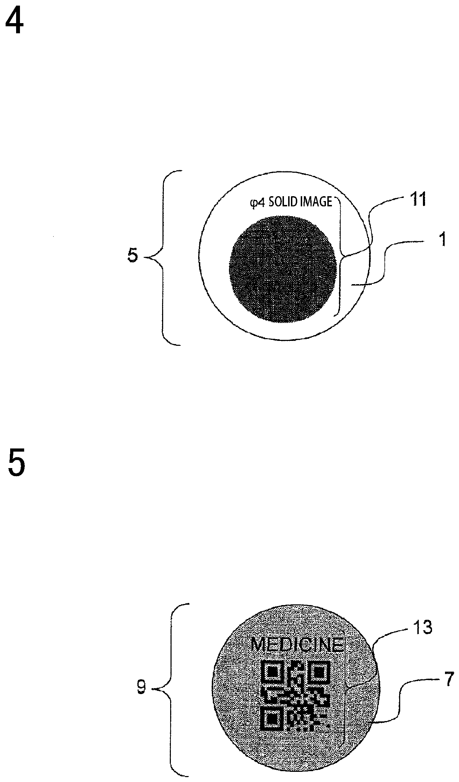

In the present embodiment, a solid image may be printed as an uncoated tablet print image 11 as shown in Fig. 4, or a two-dimensional barcode may be printed as a film-coated tablet print image 13 as shown in Fig. 5.

There is no particular limitation on the active component contained in the medical tablet. Examples of the active component include a substance that is effective for prevention and treatment of various diseases (e.g., a substance having a sleep inducing action, an tranquilizer activity, an antibacterial activity, an antihypertensive action, an anti-angina activity, an analgesic action, an anti-inflammatory activity, a tranquilizing action, a therapeutic diabetic activity, a diuretic action, an anticholinergic activity, an anti-gastric hyperactivity action, an anti-epileptic action, an ACE inhibitory activity, a β-receptor antagonist or agonist activity, an anesthesia action, an appetite suppressant action, an anti-arrhythmic action, an antidepressant action, an anticoagulant activity, an anti-diarrheal action, an antihistamine activity, an antimalarial action, an antitumor activity, an anti-tumor activity, an immunosuppressive activity, an anti-Parkinson's disease action, an anti-psychotic action, an antiplatelet activity, an antihyperlipidemic action), a substance having a cleaning action, and a substance having a scent or a deodorant action, or the like, but not limited thereto.

The tablet according to the present embodiment may as necessary contain a carrier that is acceptable for its application, together with an active component. For example, a medical tablet may contain a pharmaceutically acceptable carrier. Various kinds of pharmaceutically acceptable organic or inorganic carrier material may be used as tablet material, and an appropriate amount of excipients, lubricants, binders, disintegrating agents, thickeners or the like are blended thereto as appropriate. An additive, such as a preservative, an antioxidant, a coloring agent, and a sweetening agent, may also be used as necessary.

A tablet is illustrated with a medical tablet in the present embodiment, but the present invention is not limited thereto. There is no particular limitation on what the blue ink-jet ink according to the present embodiment is printed on. The ink may be printed on the surface of various tablets, such as a tablet to be administered to animals other than humans (e.g., pets, domestic animals, poultry), a feed tablet, fertilizer tablet, a cleaning agent tablet, a lemonade tablet, or the like. There is also no particular limitation on the size of a target object to which the blue ink-jet ink according to the present embodiment is printed, but the ink is applicable to tablets of various sizes.

(Effects of the present embodiment)

(1) The blue ink-jet ink according to the present embodiment is an edible ink-jet ink that contains Brilliant Blue FCF and New Coccine as colorants.
Such a composition improves lightfastness of an ink-jet ink while maintaining the blue print color, compared with the prior art.

(2) The Brilliant Blue FCF and New Coccine contained in the blue ink-jet ink according to the present embodiment may have a mass ratio that is in the range of 1:0.025 to 1:2.

Such a composition further improves lightfastness of an ink-jet ink while maintaining the printing color blue, compared with the prior art.

(3) The total mass of the colorants contained in the blue ink-jet ink according to the present embodiment may be in the range of 1 mass% or more and 18 mass% or less of the whole ink-jet ink.

Such a composition reliably improves lightfastness and additionally print resumability of an ink-jet ink while maintaining the printing color blue, compared with the prior art.

(4) The blue ink-jet ink according to the present embodiment may be used for direct printing on the surface of a tablet or a food product.

Such a composition makes the color tone of characters or the like directly printed on the surface of a tablet or the like less dull and more indelible, compared with the prior art.

(5) The tablet according to the present embodiment (printed uncoated tablet 5) includes a printed image 3, which is printed with the blue ink-jet ink described above.

Such a composition makes the color of the printed image 3 or the like, which is directly printed on the surface of a tablet or the like, less dull and more indelible compared with the prior art.

(6) The tablet 5 according to the present embodiment may be a medical tablet.

Such a composition makes the color of the print image 3 or the like, which is directly printed on the surface of a medical tablet, less dull and more indelible, compared with a prior art.

[Examples]

The present invention will be described in more detail by way of examples; however, the present invention is not limited to the examples.

<Example 1>

Manufacturing of uncoated and film-coated tablets printed with ink containing Blue No. 1 Colorant (Brilliant Blue FCF) and Red No. 102 Colorant (New Coccine) and durability test of the same

(Manufacturing of an ink-jet ink)

First, a printing ink was prepared. An ink-jet ink contains components, namely colorant, an organic solvent, water, resin, and a leveling agent. The first step of the preparation was to mix water with an organic solvent to produce a mixed solvent. Resin and a leveling agent was then added to the mixed solvent to produce a transparent base liquid. Finally, a colorant was added to the transparent base liquid. This is how to prepare the ink according to the present embodiment. The individual components will be described below.

The resin is material that serves as a non-volatile resin component in the ink.

The leveling agent is material that adjusts an ink surface tension so that drops ejected from an ink jet head can be properly formed. The preferable ink surface tension is in the range of 24 to 34 mN/m. Considering wettability of a tablet surface layer and permeability into the tablet; however, the surface tension value needs to be as small as possible, i.e. a highly wettable. In the present embodiment, the ink was prepared so as to have a surface tension of 24 to 28 mN/m. The surface tension value, however, should not be too small. For example, a surface tension value that is less than 24 mN/m fails to produce droplets, possibly causing faulty ejection such as a misting failure or a splash phenomenon (breakage of ejected drops).

In the present example, deionized water was used to compose the ink. Polyethylene glycol 300 (i.e. polyethylene glycol having an average molecular weight of 300), propylene glycol, glycerin, and ethanol were used as organic solvents. Deionized water, ethanol, polyethylene glycol 300, propylene glycol, and glycerin were added by the amount shown in Table 1 and stirred well to produce a mixed solvent. Polyethylene glycol 1540 (i.e. polyethylene glycol having an average molecular weight of 1540) was added to the above-mentioned mixed solvent as resin, followed by sorbitan acid fatty acid ester (product name: "Poem O-80V", Riken Vitamin Co., Ltd.) as a leveling agent, by the amount shown in Table 1 and stirred for approximately one hour to produce a transparent base liquid.

Blue No. 1 colorant and Red No. 102 colorant were added to the transparent base liquid by an amount different from each other to obtain 15 kinds of ink (1) to (15), each of which weighs 100 g. The composition of each ink is shown in Table 1.

[Table 1]

| | | Examples | | | | | | | | | | | | Comparative Examples | | |
|---|---|---|---|---|---|---|---|---|---|---|---|---|---|---|---|---|
| Inks | | Ink (1) | Ink (2) | Ink (3) | Ink (4) | Ink (5) | Ink (6) | Ink (7) | Ink (8) | Ink (9) | Ink (10) | Ink (11) | Ink (12) | Ink (13) | Ink (14) | Ink (15) |
| Mass ratio of colorants | | Blue: Red = 1:0.025 | Blue: Red = 1:0.05 | Blue: Red = 1:0.25 | Blue: Red = 1:0.43 | Blue: Red = 1:1.2 | Blue: Red = 1:2 | Blue: Red = 1:0.25 | Blue: Red = 1:0.25 | Blue: Red = 1:0.25 | Blue: Red = 1:0.25 | Blue: Red = 1:0.25 | Blue: Red = 1:0.25 | Blue: Red = 1:0 | Blue: Red = 1:3 | Blue: Red = 0:1 |
| Ink compositions (mass%) | Brilliant Blue FCF | 7.8 | 7.6 | 6.4 | 5.6 | 3.6 | 2.6 | 6.4 | 6.4 | 6.4 | 6.4 | 6.4 | 6.4 | 8 | 2 | 0 |
| | New Coccine | 0.2 | 0.4 | 1.6 | 2.4 | 4.4 | 5.4 | 1.6 | 1.6 | 1.6 | 1.6 | 1.6 | 1.6 | 0 | 6 | 8 |
| | Pure water | 51 | 51 | 51 | 51 | 51 | 51 | 45.2 | 45.2 | 45.2 | 87 | 87 | 87 | 51 | 51 | 51 |
| | Ethanol | 16.8 | 16.8 | 16.8 | 16.8 | 16.8 | 16.8 | 16.8 | 16.8 | 16.8 | 0 | 0 | 0 | 16.8 | 16.8 | 16.8 |
| | Polyethylene glycol 300 | 21 | 21 | 21 | 21 | 21 | 21 | 30 | 0 | 0 | 5 | 0 | 0 | 21 | 21 | 21 |
| | Propylene glycol | 0 | 0 | 0 | 0 | 0 | 0 | 0 | 30 | 0 | 0 | 5 | 0 | 0 | 0 | 0 |
| | Glycerin | 0 | 0 | 0 | 0 | 0 | 0 | 0 | 0 | 30 | 0 | 0 | 5 | 0 | 0 | 0 |
| | Poem O-80V | 0.2 | 0.2 | 0.2 | 0.2 | 0.2 | 0.2 | 0 | 0 | 0 | 0 | 0 | 0 | 0.2 | 0.2 | 0.2 |
| | Polyethylene glycol 1540 | 3 | 3 | 3 | 3 | 3 | 3 | 0 | 0 | 0 | 0 | 0 | 0 | 3 | 3 | 3 |
| Total mass (%) | | 100 | 100 | 100 | 100 | 100 | 100 | 100 | 100 | 100 | 100 | 100 | 100 | 100 | 100 | 100 |
| Print resumability | | Good | Good | Good | Good | Good | Good | Good | Good | Good | Good | Good | Good | Good | Good | Good |
| Fading on uncoated tablet after lightfastness test ($\Delta E$) | | 14 | 10 | 2.7 | 3.3 | 5.6 | 4.8 | 2.1 | 2.3 | 3.1 | 4.5 | 4.3 | 4.6 | 26 | 4.0 | 6.2 |
| Fading on FC tablet after lightfastness test ($\Delta E$) | | 22 | 14 | 12 | 10 | 11 | 10 | 11 | 12 | 10 | 13 | 13 | 14 | 34 | 9.3 | 22 |

| | 55 | 50 | 45 | 40 | 35 | 30 | 25 | 20 | 15 | 10 | 5 | | | |
|---|---|---|---|---|---|---|---|---|---|---|---|---|---|---|
| Blue print chromaticity (b* value) on uncoated tablet | -46.28 | -41.61 | -32.87 | -29.4 | -16.76 | -9.31 | -32.60 | -31.27 | -32.79 | -32.95 | -32.62 | -32.55 | -49.94 | -4.13 | 34.84 |
| Blue print chromaticity (b* value) on FC tablet | -39.57 | -37.11 | -29.18 | -29.38 | -16.52 | -9.63 | -29.60 | -28.27 | -29.36 | -28.87 | -28.28 | -28.08 | -43.8 | -4.96 | 32.7 |
| Blue print density (C value) on uncoated tablet | 1.42 | 1.40 | 1.34 | 1.29 | 1.19 | 1.07 | 1.34 | 1.33 | 1.34 | 1.34 | 1.34 | 1.34 | 1.46 | 0.95 | 0.17 |
| Blue print density (C value) on FC tablet | 1.00 | 1.02 | 1.02 | 1.06 | 1.04 | 0.89 | 1.02 | 1.01 | 1.02 | 1.02 | 1.02 | 1.01 | 1.00 | 0.79 | 0.12 |
| Evaluations | Good | Excellent | Excellent | Excellent | Fair | Fair | Excellent | Excellent | Excellent | Excellent | Excellent | Excellent | Poor | Poor | Poor |

Solid foreign matter in the liquid was removed by passing each above-mentioned 100 grams of ink through a membrane filter. More specifically, each ink was passed through a membrane filter (cellulose acetate film) having a pore diameter of 5.0 μm once, followed by a membrane filter (cellulose acetate film) having a pore diameter of 0.8 μm once, to obtain purified ink, each of which weighed 99 g.

(Printing)

The purified ink was used to print a circular solid image (4.0 mm in diameter) with a bending mode piezo inkjet head printer on a test uncoated tablet (based on starch) and a film-coated tablet (based on adjusted starch, coated with hydroxypropyl methylcellulose 70%, 30% mixture with titanium oxide, 6.5 mm in diameter). This resulted in an uncoated tablet and a film-coated tablet printed in each color.

(Lightfastness test)

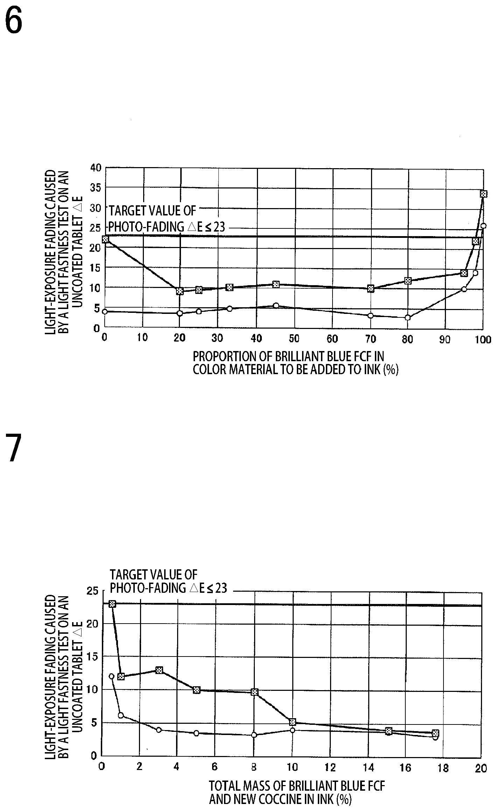

An X-Rite spectrometer "X-Rite 530", manufactured by X-RITE Inc., is placed above the solid image of a printed uncoated tablet and film-coated tablet to measure a color tone (L*a*b* color system) and an optical density of blue print (C value). These spectral densities are measured at a viewing angle of 2 degrees and under a light source D50.
The matter printed on the uncoated tablet and the film-coated tablet of which the chromaticity and optical color density were to be measured was irradiated with cumulative 1.2 million lux visible light by use of a xenon weather meter (Ci4000, Toyo Seiki Seisaku-sho, Ltd.). Measurement of chromaticity and optical color density was carried out again on the printed uncoated tablet and film-coated tablet irradiated with visible light by use of a spectrophotometer under the same conditions as before the visible light irradiation to compare the difference in the degree of change in the chromaticity and optical color density according to the mixture ratio before and after the test. The results are shown in Table 1. The measurement confirmed that a degree of fading ΔE is less than or equal to 23 relative to an ink composed solely of Blue No. 1 Colorant as long as Red No. 102 colorant is in the range of 0.025 parts by mass or more and 2 parts by mass or less relative to 1 part by mass of Blue No. 1 Colorant.
The measurement has also found that if the Red No. 102 colorant exceeds 2 parts by mass relative to 1 part by mass of Blue No. 1 colorant, the b* value exceeds -5, so that the ink assumes a red color, which is obviously inappropriate as a blue ink. In addition, if the Red No. 102 Colorant is less than 0.025 parts by mass relative to 1 part by mass of Blue No. 1 Colorant, a degree of light-exposure fading ΔE exceeds 23, which is a lightfastness standard that is evaluated as good, so that the ink tends to be less lightfast. The results are shown in Fig. 6, where the horizontal axis represents the ratio of Blue No. 1 Colorant in the colorant to be added to the ink, and the vertical axis represents a degree of light-exposure fading ΔE, which is measured by a lightfastness test of an imaged printed on an uncoated tablet and a film-coated tablet. The "o" symbol indicates that the print target is an uncoated tablet, whereas the "■" symbol indicates that the print target is a film-coated tablet. Note that ΔE = 23, which is a target value of photo-fading is shown as a bold line. In other words, if ΔE is below the solid line shown in Fig. 6, the ink is lightfast enough.
In Table 1, a vivid blue color with the lightfastness, that is, the degree of fading ΔE ≤ 14 and b* value of the printed chromaticity ≤ 20 is indicated as "Excellent", a vivid blue color with the lightfastness ΔE ≤ 23 and b* value of the printed chromaticity ≤ -20 is indicated as "Good", a vivid blue color with the lightfastness ΔE ≤ 23 and b* value of the printed chromaticity ≤ -5 is indicated as "Fair", and a vivid blue color with the lightfastness ΔE > 23 and the b* value of printed chromaticity > -5 is indicated as "Poor". If an ink is evaluated as "Excellent" on one of the uncoated tablet and the film-coated tablet and the other is evaluated as "Good", the overall evaluation on the ink is marked as "Good".

(Print resumability test)

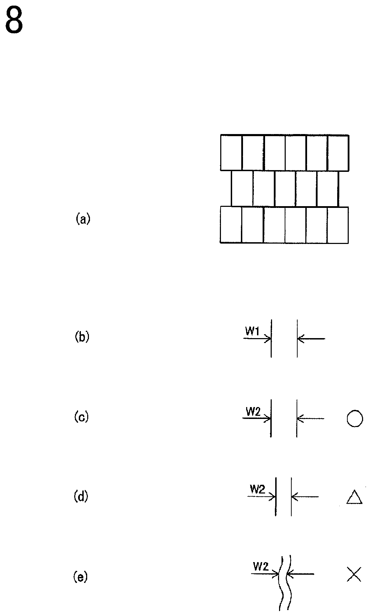

The evaluation criteria of the print resumability according to the present example will now be described below with reference to Fig. 8.
Fig. 8 is a schematic view for describing the evaluation criteria of the print resumability in the present example. Fig. 8(a) shows a test pattern used to evaluate the print resumability in the present example. This test pattern consists of straight lines extending horizontally and vertically in Fig. 8(a), each being 0.5 mm wide. Fig. 8(b) shows a test pattern width W1 immediately after ink injection. Fig. 8(c) shows a pattern width W2 immediately after printing is resumed following a 30-minute suspension. In the present example, if the pattern width W2 immediately after printing is resumed following the 30-minute suspension remains unchanged or decreases by 10 % or less compared with the pattern width W1 immediately after the ink injection, as shown in Fig. 8(c), the ink is evaluated as "Good".
In Example 2, which will be described later, the pattern width W2 immediately after printing is resumed following the 30-minute suspension decreases compared with the line width W1 immediately after the ink injection, as shown in Fig. 8(d). If the pattern width W2 decreases by less than 50 %, the ink is evaluated as "fair". Additionally, in Example 2, which will be described later, the pattern width W2 immediately after printing is resumed following the 30-minute suspension decreases in some part by 50 % or more compared with the pattern width W1 immediately after the ink injection, as shown in Fig. 8(e). In such a case, the ink is evaluated as "Poor".

<Example 2>

Manufacturing of uncoated and film-coated tablets printed with ink containing Blue No. 1 Colorant (Brilliant Blue FCF) and Red No. 102 Colorant (New Coccine) and durability test of the same

(Manufacturing of an ink-jet ink)

First, a printing ink was prepared. As with Example 1, an ink-jet ink contains components, namely colorant, an organic solvent, water, internal resin, and a leveling agent. The first step of the preparation was to mix water with an organic solvent to produce a mixed solvent. Resin and a leveling agent was then added to the mixed solvent to produce a transparent base liquid. Finally, a colorant was added to the transparent base liquid. This is how to prepare the ink according to the present embodiment. The individual components will be described below.

In the present example, deionized water was used to compose the ink. Polyethylene glycol 300 (i.e. polyethylene glycol having an average molecular weight of 300) and ethanol were used as organic solvents. Deionized water, ethanol, and polyethylene glycol 300 were added by the amount shown in Table 2 and stirred well to produce a mixed solvent.

Polyethylene glycol 1540 (i.e. polyethylene glycol having an average molecular weight of 1540) was added to the above-mentioned mixed solvent as internal resin, followed by sorbitan acid fatty acid ester (product name: "Poem O-80V", Riken Vitamin Co., Ltd.) as a leveling agent, at the amount shown in Table 2 and stirred for approximately one hour to produce a transparent base liquid.

Blue No. 1 Colorant and Red No. 102 Colorant were added to the transparent base liquid at amounts different from each other to obtain nine kinds of ink (16) to (24), each of which weighed 100 g. The composition of each ink is shown in Table 2.

[Table 2]

| Inks | | Examples | | | | | | | | |
|---|---|---|---|---|---|---|---|---|---|---|
| | | Ink (16) | Ink (17) | Ink (18) | Ink (19) | Ink (20) | Ink (21) | Ink (22) | Ink (23) | Ink (24) |
| Total amount of colorant (mass%) | | 20% | 17.5% | 15% | 10% | 8% | 5% | 3% | 1% | 0.5% |
| Ink compositions (mass%) | Brilliant Blue FCF | 14 | 12.25 | 10.5 | 7 | 5.6 | 3.5 | 2.1 | 0.7 | 0.35 |
| | New Coccine | 6 | 5.25 | 4.5 | 3 | 2.4 | 1.5 | 0.9 | 0.3 | 0.15 |
| | Pure water | 39 | 41.5 | 44 | 49 | 51 | 54 | 56 | 58 | 58.5 |
| | Ethanol | 16.8 | 16.8 | 16.8 | 16.8 | 16.8 | 16.8 | 16.8 | 16.8 | 16.8 |
| | Polyethylene glycol 300 | 21 | 21 | 21 | 21 | 21 | 21 | 21 | 21 | 21 |
| | Poem O-80V | 0.2 | 0.2 | 0.2 | 0.2 | 0.2 | 0.2 | 0.2 | 0.2 | 0.2 |
| | Polyethylene glycol 1540 | 3 | 3 | 3 | 3 | 3 | 3 | 3 | 3 | 3 |
| Total mass (%) | | 100 | 100 | 100 | 100 | 100 | 100 | 100 | 100 | 100 |
| Print resumability | | Poor | Fair | Good | Good | Good | Good | Good | Good | Good |
| Fading on uncoated tablet after lightfastness test (ΔE) | | N/A | 3.2 | 3.8 | 4.1 | 3.3 | 3.5 | 3.9 | 6.1 | 12 |
| Fading on FC tablet after lightfastness test (ΔE) | | N/A | 3.9 | 4.0 | 5.3 | 10 | 10 | 13 | 12 | 23 |
| Blue print chromaticity (b* value) on uncoated tablet | | N/A | -22.8 | -24.6 | -27.9 | -29.4 | -30.6 | -30.9 | -25.4 | -10.73 |
| Blue print chromaticity (b* value) on FC tablet | | N/A | -20.1 | -21.4 | -23.7 | -29.4 | -28.7 | -28.9 | -22.2 | -5.69 |
| Blue print density (C value) on uncoated tablet | | N/A | 1.53 | 1.49 | 1.39 | 1.29 | 1.21 | 1.05 | 0.68 | 0.36 |
| Blue print density (C value) on FC tablet | | N/A | 1.11 | 1.05 | 0.98 | 1.06 | 1.03 | 0.95 | 0.61 | 0.33 |

| Evaluations | N/A | Fair | Excellent | Excellent | Excellent | Excellent | Excellent | Excellent | Good |
|---|---|---|---|---|---|---|---|---|---|

Solid foreign matter in the liquid was removed by passing each above-mentioned 100 grams of ink through a membrane filter. More specifically, each ink was passed through a membrane filter (cellulose acetate film) having a pore diameter of 5.0 $\mu$m once, followed by a membrane filter (cellulose acetate film) having a pore diameter of 0.8 $\mu$m once, to obtain purified ink, each of which weighed 99 g.

(Printing)

The purified ink was used to print a circular solid image (4.0 mm in diameter) with a bending mode piezo inkjet head printer on a test uncoated tablet (based on starch) and a film-coated tablet (based on adjusted starch, coated with hydroxypropyl methylcellulose 70%, 30% mixture with titanium oxide, 6.5 mm in diameter). This resulted in an uncoated tablet and a film-coated tablet printed in each color.

(Lightfastness test)

An X-Rite spectrometer "X-Rite 530", manufactured by X-RITE Inc., is placed above the solid image of a printed uncoated tablet and film-coated tablet to measure a color tone (L*a*b* color system) and an optical density of blue print (C value). These spectral densities are measured at a viewing angle of 2 degrees and under a light source D50.

The matter printed on the uncoated tablet and the film-coated tablet of which the chromaticity and optical color density were to be measured was irradiated with cumulative 1.2 million lux visible light by use of a xenon weather meter (Ci4000, Toyo Seiki Seisaku-sho, Ltd.). Measurement of chromaticity and optical color density was carried out again on the printed uncoated tablet and film-coated tablet irradiated with visible light by use of a spectrophotometer under the same conditions as before the visible light irradiation to compare the difference in the degree of change in the chromaticity and optical color density according to the mixture ratio before and after the test.

The results are shown in Table 2. These results show that if the total mass of Blue No. 1 Colorant and Red No. 102 Colorant is 1 mass% or more relative to the total ink mass, the degree of change color in after a lightfastness test is $\Delta E \leq 23$, which is sufficiently lightfast as an ink-jet ink for the medical tablet. In addition, a total colorant mass exceeding 15 mass% increases ink viscosity due to increased colorant, which in turn makes it harder for the ink to be ejected from the nozzle. The ink was still printable even if the total colorant mass was 17.5 mass%, but the nozzle clogged in a print resumability test for ink ejectability carried out after a 30-minute print suspension. The case was even worse when the total colorant mass was 20 mass%, resulting in no print sample being obtained.

In other words, the results confirm that printing of the ink can be resumed sufficiently when the total colorant mass is 18 mass% or less. The results are shown in Fig. 7, where the horizontal axis represents a total mass of Blue No. 1 Colorant and Red No. 102 Colorant in the ink, and the vertical axis represents a degree of light-exposure fading $\Delta E$, which is measured by a lightfastness test of an imaged printed on an uncoated tablet and a film-coated tablet. The "o" symbol indicates that the print target is an uncoated tablet, whereas the "■" symbol indicates that the print target is a film-coated tablet. Note that $\Delta E = 23$, which is a target value of photo-fading is shown as a bold line. In Table 2, an ink that is highly print-resumable after a 30-minute print suspension is described as "Good" (see Fig. 8(c)), an ink that is not well print-resumable after a 30-minute print suspension is described as "Fair" (see Fig. 8(d)), and an ink not causing an intermittent print action but giving a poor print result and not being well print-resumable is described as "Poor" (refer to Fig. 8(e)). Note that the print pattern in Example 2 is the same as in Example 1.

Among vivid blue inks having a lightfastness of an uncoated tablet and a film-coated tablet $\Delta E \leq 23$ and print chromaticity b* value $\leq$ -20, an ink having an optical density C value of blue print $\geq$ 0.5 and having good print resumability after a 30-minute print suspension is evaluated as "Excellent", an ink having an optical density C value of blue print < 0.5 is evaluated as "Good", and an ink not having good print resumability after a 30-minute print suspension is evaluated as "Fair". An ink that is too poorly print-resumable to be printed on an uncoated tablet and a film-coated tablet is evaluated as "N/A".

The optical densities (C values) of blue print were measured by an X-Rite spectrometer "X-Rite 530", manufactured by X-Rite Inc., when a print color is expressed by combination of optical densities of cyan, magenta, and yellow.

[Reference Signs List]

    1: Tablet substrate
    3: Printed image
    5: Printed uncoated tablet
    7: Film-coated layer
    9: Printed matter on film-coated tablet
    11: Uncoated tablet print image (solid image)

13: Film-coated tablet print image (two-dimensional bar code)

**Claims**

1.  A blue ink-jet ink comprising edibility and Brilliant Blue FCF and New Coccine as colorants.

2.  The blue ink-jet ink according to claim 1, wherein a mass ratio of the Brilliant Blue FCF to the New Coccine is in a range of 1:0.025 to 1:2.

3.  The blue ink-jet ink according to any one of claims 1 and 2, wherein a total mass of the colorants is in a range of 1 mass% or more and 18 mass% or less of a whole of the ink-jet ink.

4.  The blue ink-jet ink according to any one of claims 1 to 3, wherein the blue ink-jet ink is used for direct printing onto a tablet surface and a food product.

5.  A tablet comprising a part printed with the blue ink-j et ink according to any one of claims 1 to 4.

6.  The tablet according to claim 5, wherein the tablet is a medical tablet.

1

2

3

4

φ4 SOLID IMAGE

5

11

1

5

MEDICINE

9

13

7

6

PROPORTION OF BRILLIANT BLUE FCF IN
COLOR MATERIAL TO BE ADDED TO INK (%)

7

TOTAL MASS OF BRILLIANT BLUE FCF
AND NEW COCCINE IN INK (%)

8

(a)

(b) W1

(c) W2 ○

(d) W2 △

(e) W2 ✕

## INTERNATIONAL SEARCH REPORT

| International application No. |
|---|
| PCT/JP2019/014881 |

**A. CLASSIFICATION OF SUBJECT MATTER**

Int. Cl. C09D11/328(2014.01)i, B41J2/01(2006.01)i, B41M5/00(2006.01)i, B41M5/52(2006.01)i, C09B11/26(2006.01)i, C09B29/16(2006.01)i, C09B67/46(2006.01)i

According to International Patent Classification (IPC) or to both national classification and IPC

**B. FIELDS SEARCHED**

Minimum documentation searched (classification system followed by classification symbols)

Int. Cl. C09D11/00-13/00, B41J2/01, B41M5/00, B41M5/52, C09B11/26, C09B29/16, C09B67/46

Documentation searched other than minimum documentation to the extent that such documents are included in the fields searched

Published examined utility model applications of Japan 1922-1996
Published unexamined utility model applications of Japan 1971-2019
Registered utility model specifications of Japan 1996-2019
Published registered utility model applications of Japan 1994-2019

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2015-218144 A (KISHU GIKEN KOGYO KK) 07 December 2015, paragraphs [0019], [0022], [0063]-[0066], [0071], [0073], [0090], [0092], table 2, test examples 1-3, preparation examples 4, 6, examples 10-12, 16-18 (Family: none) | 1-6 |
| Y | JP 2006-169301 A (UNION CHEMICAR CO., LTD.) 29 June 2006, paragraphs [0004], [0007], [0009], [0010], [0019], [0023], example 4, (Family: none) | 1-6 |
| Y | JP 2001-31898 A (SHIONOGI QUALICAPS KK) 06 February 2001, paragraphs [0001], [0028] (Family: none) | 1-6 |

☒ Further documents are listed in the continuation of Box C.　　☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 22.04.2019 | 14.05.2019 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2019/014881 |

C (Continuation).  DOCUMENTS CONSIDERED TO BE RELEVANT

| Category* | Citation of document, with indication, where appropriate, of the relevant passages | Relevant to claim No. |
|---|---|---|
| A | JP 9-302294 A (SAN EI GEN FFI INC.) 25 November 1997, entire text (Family: none) | 1-6 |
| A | JP 2016-8294 A (NISSHIN KASEI KK) 18 January 2016, entire text (Family: none) | 1-6 |
| A | JP 2010-47626 A (UNION CHEMICAR CO., LTD.) 04 March 2010, entire text (Family: none) | 1-6 |

Form PCT/ISA/210 (continuation of second sheet) (January 2015)

**EP 3 778 800 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006169301 A

**Non-patent literature cited in the description**

- *CHEMICAL ABSTRACTS,* 3844-45-9
- *CHEMICAL ABSTRACTS,* 2611-82-7
- *CHEMICAL ABSTRACTS,* 915-67-3
- *CHEMICAL ABSTRACTS,* 16423-68-0
- *CHEMICAL ABSTRACTS,* 25956-17-6
- *CHEMICAL ABSTRACTS,* 18472-87-7
- *CHEMICAL ABSTRACTS,* 632-69-9
- *CHEMICAL ABSTRACTS,* 3520-42-1
- *CHEMICAL ABSTRACTS,* 1934-21-0
- *CHEMICAL ABSTRACTS,* 2783-94-0
- *CHEMICAL ABSTRACTS,* 860-22-0